(19) Europäisches Patentamt
European Patent Office
Office européen des brevets

(11) **EP 1 363 615 B1**

(12) **EUROPEAN PATENT SPECIFICATION**

(45) Date of publication and mention
of the grant of the patent:
**03.01.2007 Bulletin 2007/01**

(21) Application number: **02716527.3**

(22) Date of filing: **28.01.2002**

(51) Int Cl.:
*A61K 31/19* (2006.01)        *A61K 31/14* (2006.01)
*A61P 3/00* (2006.01)        *A61P 43/00* (2006.01)

(86) International application number:
**PCT/SE2002/000141**

(87) International publication number:
**WO 2002/060429 (08.08.2002 Gazette 2002/32)**

(54) **PRESERVATION OF BODY PROTEIN**

ERHALTUNG VON KÖRPEREIGENEN PROTEINEN

CONSERVATION D'UNE PROTEINE CORPORELLE

(84) Designated Contracting States:
**AT BE CH CY DE DK ES FI FR GB GR IE IT LI LU
MC NL PT SE TR**

(30) Priority: **31.01.2001 US 773394**

(43) Date of publication of application:
**26.11.2003 Bulletin 2003/48**

(73) Proprietors:
 • **Wiklund, Lars**
  **752 36 Uppsala (SE)**
 • **Karlsson, Torbjörn**
  **757 56 Uppsala (SE)**
 • **Nordgren, Anders**
  **757 55 Uppsala (SE)**

(72) Inventors:
 • **Wiklund, Lars**
  **752 36 Uppsala (SE)**
 • **Karlsson, Torbjörn**
  **757 56 Uppsala (SE)**
 • **Nordgren, Anders**
  **757 55 Uppsala (SE)**

(74) Representative: **Johansson, Lars-Erik et al
Hynell Patenttjänst AB
Patron Carls väg 2
683 40 Hagfors / Uddeholm (SE)**

(56) References cited:
  WO-A1-87/03806        WO-A1-89/03688
  WO-A1-90/06064        WO-A1-93/23027

 • **DATABASE MEDLINE [Online] PRESTON, G.G.
  ET AL.: 'Change in subunit composition of the
  iron protein of nitrogenase from rhodospirillum
  rubrum during activation and inactivation of iron
  protein', XP002950271 Retrieved from STN
  Database accession no. 83074524 &
  BIOCHEMICAL JOURNAL vol. 205, no. 3, 01
  September 1982, pages 489 - 494**
 • **DATABASE CAPLUS [Online] DAIICHI SEIMO KK:
  'Ketoglutaric acid for controlling
  microorganisms and seaweeds in the culture
  medium of laver', XP002950270 Retrieved from
  STN Database accession no. 1995:532272 & JP 7
  053 310 A 28 February 1995**

## Description

The present invention relates to the preservation of body protein stores in patients being in a catabolic state.

BACKGROUND OF THE INVENTION

Glutamine is one of the predominant amino acids in the body and constitutes more than 50 % of the total intracellular free amino acid pool in skeletal muscle. It is utilised mainly as an energy source and nitrogen carrier. During postoperative and posttraumatic catabolism its availability is decreased. This results in depletion of skeletal muscle glutamine and, with continued utilisation of glutamine by the intestine, also to low blood glutamine levels. $\alpha$-Ketoglutarate ($\alpha$-KG), the biologic precursor of glutamine, has been tried in human enteral and parenteral nutrition. In clinical studies, parenteral and enteral administration of $\alpha$-KG was claimed to prevent severe muscle protein breakdown (1-4), and to promote mucosal repair in the small intestine (5) and wound healing (6). These recorded effects have been small and judged to be of minor importance. Therefore dipeptides of glutamine and, e.g., serine or alanine have been used instead. Glutamine turnover after trauma, sepsis or surgery is characterised by muscle protein catabolism and concomitant draining of the muscular free glutamine store to meet the increasing demand of fast dividing cells, e.g. enterocytes, immune cells and fibroblasts. During acidosis and starvation the liver participates in pH homeostasis by switching from urea to glutamine synthesis (7-9). This is achieved by a decreased periportal utilisation of bicarbonate in the urea cycle, leaving ammonium ($NH_4^+$) available for perivenous hepatic glutamine synthesis.

Ammonium is occasionally administered to patients in the form of a pharmacologically acceptable salt such as the chloride in spite of ammonium being considered neurotoxic in high concentrations is known to be neurotoxic. Therefore its recommended pharmaceutical uses are few. However, as it causes metabolic acidosis it is given by slow infusion in form of the chloride in severe metabolic alkalosis. It is however still considered to be one of the key mediators of hepatic encephalopathy (14). Tissues known to be capable of detoxifying ammonia/ammonium include skeletal muscle, the liver, and the kidneys. In skeletal muscle, about 50% of arterial ammonium content is metabolised (15, 16). The amination of $\alpha$-KG by glutamate dehydrogenase produces glutamate from which, catalysed by glutamine synthetase through the addition of one amide group, glutamine is formed. Under physiological conditions there is a hepatic uptake of glutamine which is hydrolysed through the action of periportal glutaminase to glutamate and ammonium, the latter being utilised in urea synthesis. During experimental acidosis caused by high ammonium levels, de *novo* synthesis of glutamine, catalysed by glutamine synthetase, takes place in perivenous hepatocytes (17, 18). In the kidneys, ammonium ($NH_4^+$), is liberated from its main transport form, glutamine, and excreted into the urine (19).

The administration of glutamine and/or small peptides comprising glutamine residues to a patient being in a state of glutamine depletion thus is a less than direct way of coping with such deficiency since glutamine is poorly soluble water and cannot be sterilised by autoclavation while dipeptides are costly. A more direct way of preserving or raising blood glutamine levels thus is highly desirable.

OBJECTS OF THE INVENTION

It is an object of the present invention to provide a means for preserving body protein stores in a patient being in a catabolic state by inducing endogenous synthesis of glutamine.

It is another object of the present invention to provide a means for preserving body protein stores in a patient being in a catabolic state by inducing endogenous synthesis of arginine.

It is a further object of the present invention to provide a means for inducing such endogenous synthesis in a manner such as to avoid exerting an extra metabolic strain on the patient.

Additional objects of the invention will become apparent from the following summary of the invention, the description of preferred embodiments thereof, and the appended claims.

SUMMARY OF THE INVENTION

The present invention is based on the hypothesis that combined administration of $\alpha$-ketoglutaric acid and ammonium ion to a catabolic patient protects bodily protein stores and especially muscle protein from breakdown, that is, from being used as a source of free glutamine. The term "catabolic state" includes conditions in which glutamine stores are depleted or at risk of being depleted, such as in a patient in intensive care, etc. The term "catabolic state" also includes conditions in which arginine stores are depleted or at risk of being depleted, such as in a patient in intensive care, etc.

This hypothesis was tested in an anaesthetised piglet model to which a combination of $\alpha$-ketoglutaric acid and ammonium ion were given by intravenous infusion. The term "in combination" here refers to the administration of $\alpha$-ketoglutaric acid and ammonium in temporal dependence of each other, the one or the other or both being administered continuously or intermittently.

The synthesis of glutamine/protection against protein breakdown in the animal model was assessed by measuring plasma glutamine concentration and glutamine release from hind leg skeletal muscle and the splanchnic area. In this "minor trauma" model, the effects of different dosages of intravenously administered $\alpha$-KGA and ammonium chloride on plasma concentration and splanchnic and hind leg skeletal muscle turnover of glutamine, glutamate, alanine, arginine, urea and ammonium were investigated.

Although numerous formulations of varying composition containing $\alpha$-KG are disclosed in the art to be useful in preserving protein stores in a catabolic state, the present inventors have not found any reports on the combined effects of $\alpha$-KG and/or $\alpha$-KGA and ammonium ion on the important amide/ ammonium source glutamine.

The present inventors investigated the dose-response effects of intravenous administration of $\alpha$-KGA and ammonium, especially the immediate effects on the splanchnic and skeletal muscle turnover as well as plasma concentrations of glutamine, glutamate, alanine, arginine and ammonium.

Thus, according to the present invention, is disclosed the use of a pair of pharmaceutical agents selected from (a) at least one of $\alpha$-ketoglutarate and $\alpha$-ketoglutaric acid and (b) ammonium, the amounts of the pair being effective to preserve skeletal muscle for the manufacture of a medicament for their concomitant administration in separate pharmaceutically acceptable carriers for preserving bodily protein stores in a catabolic patient.

In the aforementioned animal model the present inventors found that infusion of $\alpha$-KGA and ammonium increased arterial glutamine concentration dose-dependently when the ammonium load was increased and the dose rate of $\alpha$-KGA was kept constant (Group 2) but not if the dose rate of $\alpha$-KGA was increased and the ammonium dosage was held constant (Group 1).

Thus, in the use of the invention, it is preferred to conduct the administration of $\alpha$-KG and/or $\alpha$-KGA and ammonium concomitantly.

According to the invention it is also preferred for the concomitant administration of (a) $\alpha$-KG or $\alpha$-KGA and (b) ammonium to last for more than one hour, more preferred for more than 6 hours but less than 36h.

According to a preferred aspect of the invention, said use is in respect of a patient having undergone accidental trauma or surgery, intermittently or continuously, during a substantial portion of the posttraumatic/postoperative period in which he is in a catabolic state, such as during three days or more.

A preferred constant dosing rate of $\alpha$-KG or $\alpha$-KGA in the use of the invention is from about 0.5 $\mu$mol·kg$^{-1}$ ·min$^{-1}$ to about 15 $\mu$mol·kg$^{-1}$ ·min$^{-1}$.

A preferred constant dosing rate of $NH_4^+$ in the use of the invention is from about 1 $\mu$mol·kg$^{-1}$ ·min$^{-1}$ to about 10 $\mu$mol·kg$^{-1}$ ·min$^{-1}$, the increase over a period of administration being preferably by a factor of from 2 to 5.

According to the present invention is also disclosed a pharmaceutical dosage unit comprising a first pharmaceutical composition comprising at least one of $\alpha$-ketoglutarate and $\alpha$-ketoglutaric acid in a pharmaceutically acceptable carrier and a second pharmaceutical composition comprising ammonium in a pharmaceutically acceptable carrier, the total amount of the at least one of $\alpha$-ketoglutarate and $\alpha$-ketoglutaric acid and the ammonium being effective to preserve skeletal muscle.

It was surprisingly found that the elevated arterial glutamine concentration was not associated with an increased release of glutamine from skeletal muscle or the splanchnic region, suggesting other tissues to be responsible for the increased plasma concentration of glutamine.

The experimental model used in this study represents a minor trauma inasmuch as the surgical interventions was limited to a few small incisions of the skin and the tissue damage therefore was kept to a minimum added to the stress of being anaesthetised. This being so, it can be assumed that the experimental set-up did not induce alterations in plasma concentration of amino acids otherwise seen after major (but not minor) surgery (11). The experimental period was short, only four hours, and it may be argued that it was too short for changes in the metabolism of proteins to manifest itself, but earlier reports (11, 12) have shown decreased plasma concentration of glutamine as early as 1½ -2½ hours after skin incision in patients undergoing major surgery.

Our wish to elucidate the effects of the substrate administration under non-trauma conditions refrained us from including kidney and liver turnovers in the present study. The baseline measurements of hemodynamic and respiratory variables were found to be within the normal range (13) compared to conscious piglets of this size and age.

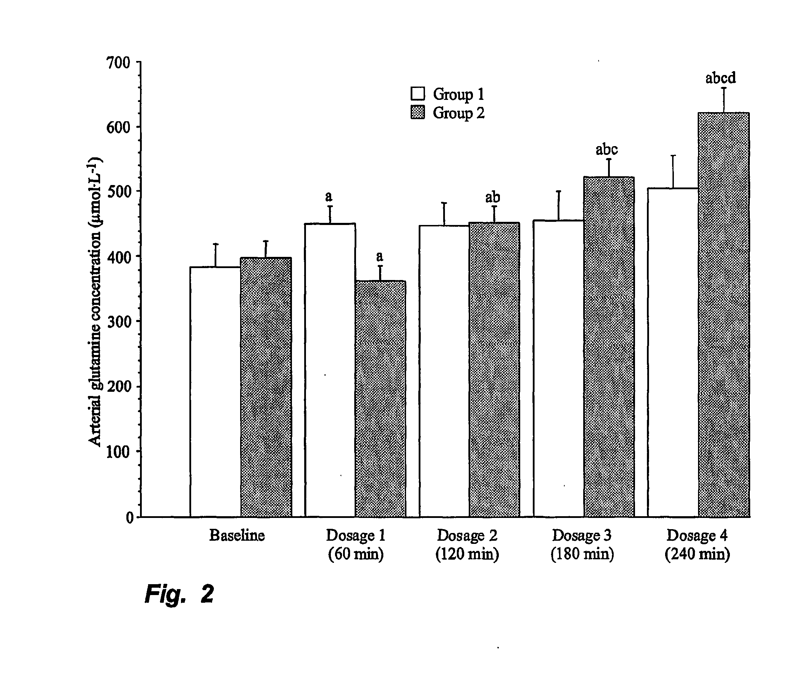

Significant effects on the plasma concentration of the amino acids under study were observed. The arterial glutamine concentration in Group 1 and Group 2 demonstrated different development (Fig. 2). After 60 minutes, that is after one hour of ammonium chloride infusion (Group 1), or after one hour of $\alpha$-KGA infusion (Group 2), the glutamine level was increased in Group 1 but decreased in Group 2. The increase in Group 1 can be attributed to the normal response of the liver to enhance glutamine synthesis when the ammonium load is increasing. Since we studied the overall splanchnic turnover only this could not be verified by our data. The increase after 60 minutes was however transient. For the rest of the experimental period there were no differences compared to the baseline glutamine level. This demonstrates that added $\alpha$-KGA in increasing dosages has no effect on arterial concentration of glutamine. This is in line with the findings of other investigators (21), but it does not rule out beneficial effects of $\alpha$-KG or $\alpha$-KGA

supplementation. In a review article, Cynober (22) suggests that $\alpha$-KG preserves endogenous glutamine stores, which may explain the non-existence of increased release of glutamine from skeletal muscle observed in the present study.

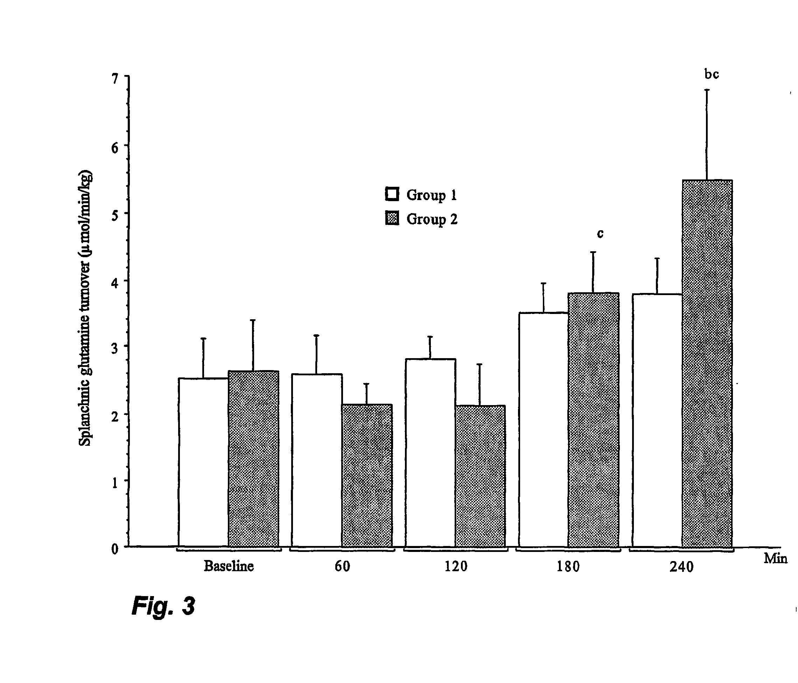

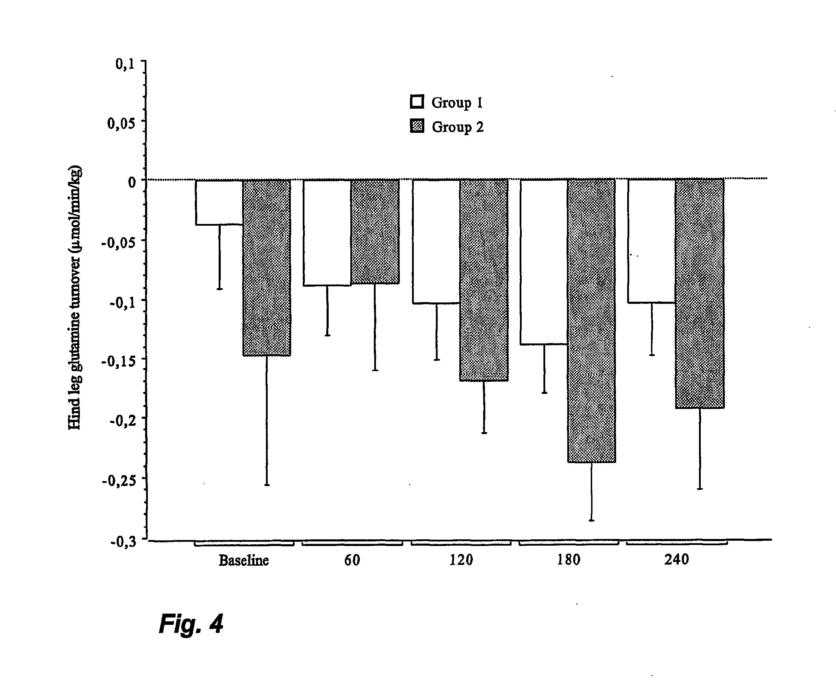

In Group 2, glutamine concentration dropped $9 \pm 2$ % after one hour of $\alpha$-KGA infusion compared to the baseline level, a fact that cannot be readily explained, while commencement of ammonium chloride administration caused arterial levels of glutamine to increase in a dose-dependent manner by $73 \pm 8$ % compared to the lowest value (60 min). Hind leg exchange data (Fig. 4) do not explain this increase. Although hind leg arterio-venous differences for glutamine did increase, there was no increased net release of glutamine, explicable by a decreased femoral artery blood flow. The reduced hind leg blood flow was not related to lower cardiac output but probably the effect of a redistribution of cardiac output. Data for splanchnic blood flow support this explanation, at least in Group 2, but only partially in Group 1. The fact that the splanchnic glutamine uptake increases in Group 2 during the last two hours of substrate infusion (Fig. 3) indicates a possibility that the liver may not, at least not alone, be responsible for the elevated glutamine levels. It is, however, possible that an increased net splanchnic regional uptake could conceal an increased release by the liver by a quantitatively larger uptake by the intestines. In support of such an interpretation it may be noted that hepatic vein glutamine concentration data indicate an increased synthesis. Furthermore, both arterial and hepatic venous urea concentration was increased in Group 2, disclosing an enhanced urea synthesis. This circumstance is in accordance with the known zonal localisation of the hepatocytes (23), with periportal hepatocytes containing urea cycle enzymes at the inflow, and the perivenous hepatocytes acting as scavenger cells for ammonium, at the outflow from the liver acinus.

Plasma concentration of glutamate in Group 1 was, like glutamine, unaffected by the substrate infusion and in Group 2 there was an increase after 120 min, which was maintained for the duration of the experiment, compared to baseline. A possible interpretation of these results is that the glutamate concentration is controlled by a continuous amidation to glutamine. Turnover data for glutamate does not preclude such an interpretation.

Alanine is a gluconeogenic amino acid and, as could be expected, plasma concentration of alanine decreased in both Groups in order to maintain blood glucose levels.

Some mention of the fate of parenterally administered ammonium is in place. Normally, enteral ammonium is produced from the digestion of proteins and bacterial hydrolysis of urea. It is absorbed by the gut and after reaching the portal circulation, it is efficiently removed by the liver. Some of the parenterally administered ammonium, on the other hand, escapes the detoxifying action of the liver, and contributes to higher plasma concentration.

In both Groups, the splanchnic ammonium uptake mirrored the delivered ammonium dosage. Skeletal muscle uptake, however, presented some unexpected findings in Group 1. After 60 minutes of ammonium infusion, a maximum uptake was reached only to be followed by a continuous decrease with gradually increased $\alpha$-KGA dosages. The reason for this is unclear, but it might be due to $\alpha$-KG, above a threshold concentration, counteracting ammonium uptake.

The invention will be more fully understood by studying a detailed description of a number of preferred embodiments illustrated by a number of Figures.

SHORT DESCRIPTION OF THE FIGURES

Fig. 1    is a timeline for the $NH_4^+$/$\alpha$-KGA administration protocol in which $NH_4^+$ is the mean value for Groups 1 and 2 of experimental animals.

Fig. 2    is a diagram illustrating arterial glutamine concentration for Groups 1 and 2. Statistical significance ($p<0.05$) according to the Wilcoxon signed rank test is indicated by "a"-different from baseline; "b"-different from 60 min; "c"-different from 120 min: "d"-different from 180 min, in each group respectively.

Fig. 3    is a diagram illustrating splanchnic glutamine turnover for Groups 1 and 2. Positive values (mean $\pm$SEM) indicate uptake. Statistical significance ($p<0.05$) according to the Wilcoxon signed rank test is indicated by "b"-different from 60 min; "c"-different from 120 min.

Fig. 4    is a diagram illustrating hind leg glutamine turnover for Groups 1 and 2. Negative values (mean $\pm$ SEM) indicate release.

DESCRIPTION OF PREFERRED EMBODIMENTS

**EXAMPLE 1. Experimental animals**. Sixteen piglets of a mixed breed (Hampshire, Yorkshire and Swedish land race), of both sexes, weighing 20.9 to 33.2 kg (mean weight 25.6 kg), were used in this study. Food was withheld from the animals for twelve hours prior to the experiment, but they had free access to water.

**EXAMPLE 2. Anaesthesia** was induced by an intramuscular injection of xylazine 2.2 mg·kg$^{-1}$, tiletamine 3 mg·kg$^{-1}$ + zolazepam 3 mg·kg$^{-1}$ and atropine 0.04 mg·kg$^{-1}$. In addition, all animals were given an intravenous bolus of morphine 1 mg·kg$^{-1}$ and, following tracheotomy, pancuronium bromide 0.3 mg·kg$^{-1}$. For continuation, an infusion of sodium pentobarbital 8 mg·kg$^{-1}$·h$^{-1}$ and pancuronium bromide 0.26 mg·kg$^{-1}$·h$^{-1}$ was started immediately after induction.

During the experiment, the animals received 10 mL·kg$^{-1}$ of dextran 70 and 18 mL·kg$^{-1}$·h$^{-1}$ of isotonic saline to maintain normovolaemia. After the last measurements, still under anaesthesia, the animals were killed with an overdose of potassium chloride. After induction of anaesthesia the animals were tracheotomised and ventilated through an 8 mm endotracheal tube (Mallincrodt Laboratories, Athlone, Ireland) with a Servo ventilator 900C (Siemens-Elema, Solna, Sweden). Ventilation was maintained at 25 breaths per minute with a positive end-expiratory pressure of 5 cm of H$_2$O. Tidal volume was adjusted to keep arterial PCO$_2$ between 5.0 and 6.0 kPa. The inspired gas mixture during the surgical procedures was 30 % oxygen in nitrous oxide and 30 % oxygen in air during the experimental period. The body temperature was kept stable by means of a thermostat controlled heating pad and warmed intravenous infusions. After skin incisions, a catheter was introduced in the left external jugular vein and advanced to the right hepatic vein under fluoroscopic control. Its position was confirmed by a contrast injection. The right external jugular vein was used for placement of a central venous catheter and a 7F pulmonary artery thermodilution catheter. An arterial catheter was inserted in a subclavian branch and advanced to a central position. Through a small suprapubic incision, a urinary catheter was placed in the bladder. A cutdown was made to place an ultrasonic flow probe (Transonic Systems Inc., Ithaca, N.Y., USA) around the femoral artery and to cannulate the femoral vein for blood sampling.

**EXAMPLE 3. Blood analyses and other measurements.** Arterial blood gases were analysed on an ABL300/OSM3 system (Radiometer, Copenhagen, Denmark). Amino acid concentration were analysed in arterial, hepatic and femoral venous plasma on a Pharmacia LKB Biochrom 20 Amino Acid Analyser (Pharmacia, Uppsala, Sweden), using continuous flow ion exchange chromatography. The column eluate was mixed with ninhydrin reagent for amino acid detection. Arterial and venous plasma urea and ammonium was analysed with routine enzymatic methods on a Hitachi 717 Automatic Analyser (Hitachi Ltd., Tokyo, Japan). Blood glucose was analysed on a Reflolux® II (Boehringer Mannheim, Germany), using the glucose-oxidase/peroxidase reaction. Arterial and hepatic venous blood was drawn for calculations of splanchnic blood flow. ECG, arterial and central venous pressure and cardiac output, were monitored and displayed on a Sirecust 1281 (Siemens Medical Electronics Inc., Danvers, MA, USA). Cardiac output was measured with the thermodilution technique using 10 mL of iced normal saline as indicator. The mean value of at least three measurements was adopted.

**EXAMPLE 4. Calculation of splanchnic blood flow** was carried out according to the constant dye infusion technique (10). The dye, indocyanine green (PULSION, Medical Systems, Munich, Germany), was given intravenously at a rate of 0.17 mg·min$^{-1}$, which gave a stable arterial concentration of indocyanine green. Arterial and hepatic venous blood was drawn simultaneously and after centrifugation at 3000 rpm for 20 min, the indocyanine green plasma concentration was determined spectrophotometrically (Hitachi 101, Hitachi Ltd., Tokyo, Japan) at a wavelength of 805 nm. Splanchnic plasma flow was calculated according to a formula derived from Fick's principle:

$$F_P = \frac{I}{(C_a - C_v)}$$

Arterial blood hematocrit (Erythrocyte Volume Fraction, EVF) was measured and the value added to the formula in order to calculate splanchnic blood flow:

$$F_B = \frac{I}{(C_a - C_v)(1 - EVF)}$$

where $F_P$ = splanchnic plasma flow (mL·min$^{-1}$), $F_B$ = splanchnic blood flow (mL·min$^{-1}$), $I$ = indocyanine green infusion rate (mg·min$^{-1}$), $C_\alpha$ = arterial plasma concentration of indocyanine green (mg·mL$^{-1}$), $C_v$ = hepatic venous plasma concentration of indocyanine green (mg·mL$^{-1}$). Femoral artery plasma flow was calculated as blood flow · (1-EVF). The turnover of amino acids, ammonium and urea were calculated as plasma flow times the arterio-venous plasma concentration difference with negative values indicating release and positive values uptake.

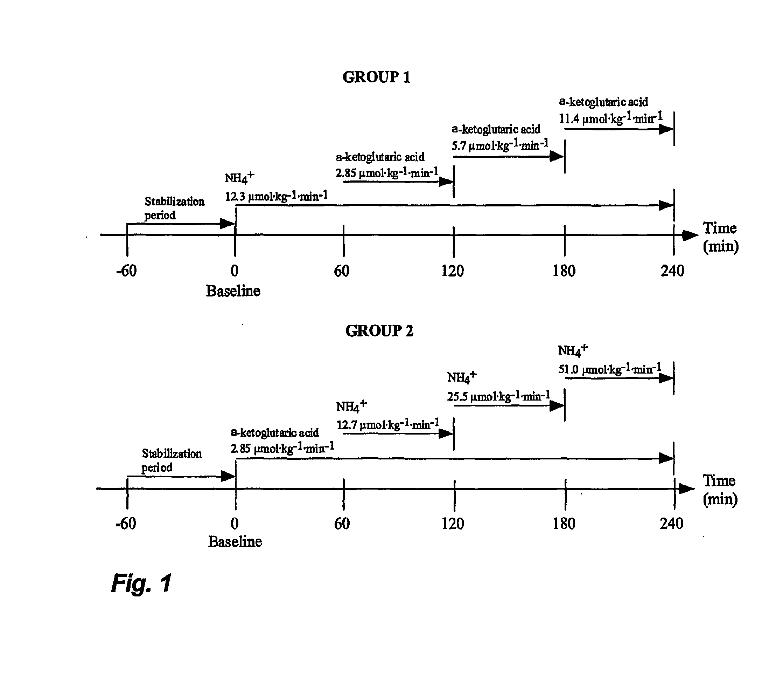

**EXAMPLE 5. Experimental protocol.** After anaesthesia and surgical preparation the piglets were allowed a stabilisation period of one hour and randomly assigned to either Group 1 or Group 2. Blood sampling, measurements and pressure readings were made at 0 min (baseline), and at four different dosages in each Group, after 60 (dosage 1), 120 (dosage 2), 180 (dosage 3) and 240 min (dosage 4). The timeline and interventions of the experiment is shown in Fig. 1. It should be pointed out that during the first hour of the study period ammonium chloride alone was administered in Group 1 and α-KGA alone in Group 2. The basal infusion rate of ammonium, aiming at a base excess (BE) of -6 mmol·L$^{-1}$ at the end of the experiment, was calculated according to the formula: NH$_4$$^+$ (mmol) = 0.3·(BE+(-6))·kg body weight, where BE was the arterial base excess value at the baseline measurements.

Group 1: Eight animals receiving an infusion of $NH_4Cl$ mixed with normal saline, at a constant rate of 12.3 $\mu$mol·kg$^{-1}$·min$^{-1}$ for 240 minutes, commencing after the baseline measurements (0 min). An infusion of $\alpha$-KGA (Sigma Chemical Co, St Louis, MO, USA), dissolved normal saline, was started after the 60 min measurement, at a rate of 2.85 $\mu$mol·kg$^{-1}$·min$^{-1}$ during the first (60-120 min), 5.7 $\mu$mol·kg$^{-1}$·min$^{-1}$ during the second (120-180 min) and 11.4$\mu$ mol·kg$^{-1}$·min$^{-1}$ during the third (180-240 min) hour of infusion.

Group 2: Eight animals receiving a constant infusion of $\alpha$-KGA at a rate of 2.85 $\mu$mol·kg$^{-1}$·min$^{-1}$ for 240 minutes, commencing after the baseline measurements (0 min) and an infusion of $NH_4Cl$ started after the 60 min measurement. During the first hour of infusion (60-120 min) the rate was 12.7 $\mu$mol·min$^{-1}$·kg$^{-1}$, 25.5 $\mu$mol·kg$^{-1}$·min$^{-1}$ during the second hour (120-180 min) and 51.0 $\mu$mol·kg$^{-1}$·min$^{-1}$ during the last hour (180-240 min) of infusion.

**EXAMPLE 6. Statistics.** The data are presented as mean $\pm$ SEM. Due to the small sample sizes in the study Groups we did not assume that the data were normally distributed. We therefore used nonparametric statistical tests. The Wilcoxon signed rank test was used for paired comparisons within each Group. Correlation between variables was tested with the Spearman rank correlation coefficient test. Differences were considered statistically significant if $p < 0.05$. The statistical calculations were performed with StatView® 5.0 (SAS Institute Inc., Cary, NC, USA) computer software.

**EXAMPLE 7. Blood gases and haemodynamics**. Blood gas and hemodynamic variables are presented in Table 1.1 and 1.2. In Group 1 arterial pH decreased from baseline 7.46 $\pm$ 0.01 to 7.30 $\pm$ 0.01 ($p = 0.012$) at the end of the experiment, and in Group 2 from 7.47 $\pm$ 0.004 to 7.29 $\pm$ 0.01 ($p = 0.012$). Baseline arterial $PCO_2$ in Group 1 was 5.28 $\pm$ 0.07 kPa and 5.31 $\pm$ 0.06 kPa in Group 2 and normoventilation was maintained for the whole experimental period. In Group 1, baseline cardiac output (C.O.) was 140 $\pm$ 10 mL-min$^{-1}$-kg$^{-1}$, and splanchnic blood flow 51 $\pm$ 7 mL·min$^{-1}$·kg$^{-1}$, and it did not change significantly. In Group 2, however, C.O. decreased after 60 min compared to the baseline ($p = 0.025$) C.O. of 157 $\pm$ 13 mL·min$^{-1}$·kg$^{-1}$, after which it resumed the baseline level. In Group 2, the baseline splanchnic blood flow of 53 $\pm$ 7 mL·min$^{-1}$·kg$^{-1}$ was stable until the 180 min measurement when there was an increase ($p = 0.017$) which was not present at the last measurement. In Group 1, baseline femoral artery blood flow was 6.0 $\pm$ 0.4 mL·min$^{-1}$·kg$^{-1}$ and there was a significant decrease to 4.7 $\pm$ 0.4 mL·min$^{-1}$·kg$^{-1}$ after 60 min ($p = 0.018$) and it continued to decrease until 120 min ($p = 0.012$) at which level it remained during the rest of the experiment. In Group 2 the baseline value was 7.4 $\pm$ 0.8 mL·min$^{-1}$·kg$^{-1}$ and there was a decrease after 120 min ($p = 0.012$) which, as in Group 1, remained stable onwards to the end of the experiment.

**EXAMPLE 8. Amino acids, ammonium, urea and glucose.** Arterial and venous concentration data are shown in Table 2.1 and 2.2

Glutamine. In Group 1, the arterial glutamine concentration was increased after 60 min ($p = 0.012$) compared to 384 $\pm$ 34 $\mu$mol·L$^{-1}$ at baseline. Glutamine concentration at the other dosages were not significantly different from baseline. In Group 2 the baseline value was 397 $\pm$ 26 $\mu$mol·L$^{-1}$ and an initial decrease was seen after 60 min ($p = 0.017$), and subsequently there were stepwise increasing glutamine concentrations for each dosage increment. Interestingly, in Group 2, there was a significant correlation ($r_s = -0.77$; $p < 0.0001$) between arterial glutamine concentration and arterial pH. No such correlation was seen in Group 1 ($r_s = -0.28$; $p = 0.089$).

Glutamate. In Group 1 the initial arterial concentration of glutamate was 181 $\pm$ 18 $\mu$mol·L$^{-1}$ and an increase was observed at 180 min ($p = 0.036$) compared to the baseline value. In Group 2 there was an elevated glutamate concentration after 120, 180 and 240 min compared to the initial 241 $\pm$ 23 $\mu$mol·L$^{-1}$.

Alanine. The arterial alanine concentration in Group 1 was significantly lower ($p < 0.05$) at all the studied dosages compared to the baseline value (503 $\pm$ 51 $\mu$mol·L$^{-1}$), and at 60 min compared to 240 min. In Group 2 there was a significant decrease after 60, 180 and 240 min compared to 506 $\pm$ 38 $\mu$mol·L$^{-1}$ at baseline, and also at 240 min ($p < 0.05$) compared to 60 and 180 min.

Arginine. There was a marked increase in arterial arginine concentration in both Groups. From 71 $\pm$ 6 $\mu$mol·L$^{-1}$ to 127 $\pm$ 8 $\mu$mol·L$^{-1}$ (+ 44 %, $p = 0.012$) in Group 1 and 77 $\pm$ 8 $\mu$mol·L$^{-1}$ to 128 $\pm$ 6 $\mu$mol·L$^{-1}$ (+ 40 %, $p = 0.017$) in Group 2. $\alpha$-Ketoglutarate is a precursor of arginine and may as such be responsible for the 44% (Group 1) and 40% (Group 2) increase in arterial arginine concentration. Also, it was reported in a human study by Reaich et al. (20) that ammonium chloride induced acidosis was found to increase the plasma level of arginine and other amino acids but, in contrast to what is disclosed here, not of glutamine. There was a slight tendency for splanchnic uptake of arginine to switch to a net release after 180 min in Group 2 while in Group 1 no apparent effect on splanchnic turnover was observed. Hind leg exchange data was similar to that of the splanchnic bed with a significant switch to release after 180 min that was not present at the final measurement. Again, there was no effect on the hind leg uptake of arginine in Group 1.

Ammonium. The arterial ammonium concentration in Group 1, 36 $\pm$ 2 $\mu$mol·L$^{-1}$, increased after 60 min infusion to a level which was maintained for the rest of the study period. The baseline ammonium concentration in Group 2 was 41 $\pm$ 4 $\mu$mol·L$^{-1}$. The ammonium infusion started after the 60 min measurement and there was an increase after each change of the dose rate.

Urea. In Group 1 the arterial urea concentration did not change during the experiment, compared to 3.4 $\pm$ 0.4 mmol·L$^{-1}$ at baseline. In Group 2 the arterial urea concentration at baseline, 2.9 $\pm$ 0.2 mmol·L$^{-1}$, was increased to 4.3 $\pm$ 0.2 ($p = 0.012$) after 240 min. Compared to the 60 min value, the point when the ammonium infusion was started, the urea concentration increased with every new dosage.

Glucose. The baseline blood glucose level was $6.0 \pm 0.4$ and $5.7 \pm 0.5$ mmol·L$^{-1}$ in Group 1 and Group 2, respectively. In Group 1 there was a decrease after 180 and 240 min compared to baseline, and in Group 2 there were no changes compared to baseline but a decrease at 180 and 240 min compared to the 60 min value.

Amino acid, ammonium and urea turnover. Values are shown in Table 3.1 and 3.2. At baseline, splanchnic glutamine uptake was $2.5 \pm 0.6$ µmol·min$^{-1}$·kg$^{-1}$ in Group 1, and it was not affected by the different dose levels of the substrate infusion. In Group 2 there was a splanchnic uptake at baseline of $2.6 \pm 0.8$ µmol·min$^{-1}$·kg$^{-1}$ which was not significantly changed by the different dose rates. There was, however, a higher uptake after 180 ($p = 0.017$) and 240 min ($p = 0.012$) compared to the uptake at 120 min and at 240 min compared to 60 min. From hind leg skeletal muscle there was a net release of glutamine in both Groups which was not affected by the different dose rates.

Glutamate turnover. There was a splanchnic release of $5.04 \pm 0.49$ µmol·min$^{-1}$·kg$^{-1}$ glutamate in Group 1 at baseline. The glutamate release at 240 min was significantly lower than was seen with all other dosages. In Group 2, the different dosages did not change the baseline glutamate release of $6.58 \pm 0.52$ µmol·min$^{-1}$·kg$^{-1}$. Skeletal muscle turnover of glutamate at baseline presented a net uptake of $0.19 \pm 0.03$ µmol·min$^{-1}$·kg$^{-1}$ in Group 1 and $0.30 \pm 0.05$ µmol·min$^{-1}$·kg$^{-1}$ in Group 2. In Group 1, the different dosages did not change the glutamate turnover, and in Group 2 there was a greater uptake after 120 min compared to 180 min, but no differences compared to baseline.

Alanine turnover. In both Groups, there was a splanchnic uptake of alanine at baseline, $3.46 \pm 0.89$ and $4.24 \pm 1.14$ µmol·min$^{-1}$·kg$^{-1}$ in Group 1 and 2, respectively, and it remained unaltered during the experimental period. In skeletal muscle there was a release of alanine, $0.19 \pm 0.03$ and $0.27 \pm 0.13$ µmol·min$^{-1}$·kg$^{-1}$, respectively, in Group 1 and 2. It was not altered by the different dose levels.

Arginine turnover. There was a splanchnic uptake of arginine in both Groups, $0.06 \pm 0.21$ µmol·min$^{-1}$·kg$^{-1}$ and $0.39 \pm 0.36$ µmol·min$^{-1}$·kg$^{-1}$, respectively, at baseline; no significant changes occurred during the study period. Hind leg uptake of arginine was $0.01 \pm 0.03$ µmol·min$^{-1}$·kg$^{-1}$ in Group 1 and $0.09 \pm 0.04$ µmol·min$^{-1}$·kg$^{-1}$ in Group 2. No effect on hind leg uptake was observed in Group 1 whereas in Group 2 a switch from uptake to release was seen after 180 min ($p < 0.05$).

Urea turnover. Group 1 demonstrated a net release of urea from the splanchnic region, and the baseline level $3.2 \pm 1.3$ µmol·min$^{-1}$·kg$^{-1}$, was not significantly changed during the study period. At 180 min there was, however, an increased release compared to the previous dose level ($p = 0.028$). In Group 2 there was a release of $6.0 \pm 3.2$ µmol·min$^{-1}$·kg$^{-1}$ at baseline and no significant changes in splanchnic urea turnover compared to the baseline condition were recorded.

Ammonium turnover. At baseline there was a small splanchnic uptake of ammonium in both Groups, $0.52 \pm 0.20$ µmol·min$^{-1}$·kg$^{-1}$ and $0.53 \pm 0.22$ µmol·min$^{-1}$·kg$^{-1}$, in Group 1 and 2, respectively. In Group 1 there was a significant increase after 60 min which was maintained for the duration of the study period with a peak splanchnic uptake of $4.1 \pm 1.0$ µmol·min$^{-1}$·kg$^{-1}$ after 180 min. Group 2 presented an increased uptake after 120 min and for each dosage there was a concomitant increase in the splanchnic uptake. Hind leg ammonium turnover in Group 1 was near zero at baseline with only a small uptake of $0.02 \pm 0.03$ µmol·min$^{-1}$·kg$^{-1}$ which was significantly increased after 60, 120 and 180 min and then decreased to almost baseline level, $0.05 \pm 0.09$ µmol·min$^{-1}$·kg$^{-1}$, after 240 min. Hind leg turnover in Group 2 was similar to Group 1 at baseline and it was significantly increased after 120 ($p = 0.012$) and 240 ($p = 0.012$) minutes compared to the baseline value. Compared to the 60 min measurement ammonium uptake was higher at all of the ensuing dosages. Significantly higher uptake was also seen at 240 min compared to the 120 min measurement.

References

1. Wernerman J, Hammarqvist F, Vinnars E. Alpha-ketoglutarate and postoperative muscle catabolism. Lancet 1990;335(8691):701-3.
2. Vinnars E, Hammarqvist F, von der Decken A, Wernerman J. Role of glutamine and its analogs in posttraumatic muscle protein and amino acid metabolism. JPEN J Parenter Enteral Nutr 1990;14(4 Suppl):125S-129S.
3. Le Bricon T, Cynober L, Baracos VE. Ornithine alpha-ketoglutarate limits muscle protein breakdown without stimulating tumor growth in rats bearing Yoshida ascites hepatoma. Metabolism: Clinical And Experimental 1994; 43(7):899-905.
4. Blomqvist BI, Hammarqvist F, von der Decken A, Wernerman J. Glutamine and alpha-ketoglutarate prevent the decrease in muscle free glutamine concentration and influence protein synthesis after total hip replacement. Metabolism: Clinical And Experimental 1995;44(9):1215-22.
5. Duranton B, Schleiffer R, Gosse F, Raul F. Preventive administration of ornithine alpha-ketoglutarate improves intestinal mucosal repair after transient ischemia in rats. Crit Care Med 1998;26(1):120-5.
6. De Bandt JP, Coudray-Lucas C, Lioret N et al. A randomized controlled trial of the influence of the mode of enteral ornithine alpha-ketoglutarate administration in burn patients. Journal Of Nutrition 1998;128(3):563-9.
7. Atkinson D, Bourke E. The role of ureagenesis in pH homeostasis. Trends Biochem Sci 1984;July:297-300.

8. Häussinger D, Meijer A, Gerok W, Sies H. Hepatic nitrogen metabolism and acid-base homeostasis. In: Häussinger D, editor. pH homeostasis: Mechanisms and control. London: Academic Press Ltd; 1988. p. 337-377.

9. Wiklund L. Carbon dioxide formation and elimination in man. Recent theories and possible consequences. Upsala Journal Of Medical Sciences 1996;101(1):35-67.

10. Bradley S, Ingelfinger F, Bradley G, Curry J. The estimation of hepatic blood flow in man. J Clin Invest 1945; 24:890-897.

11. Parry-Billings M, Baigrie RJ, Lamont PM, Morris PJ, Newsholme EA. Effects of major and minor surgery on plasma glutamine and cytokine levels. Arch Surg 1992;127(10):1237-40.

12. Lund J, Stjernström H, Bergholm U et al. The exchange of blood-borne amino acids in the leg during abdominal surgical trauma: effects of glucose infusion. clinical science 1986;71(5):487-96.

13. Hannon JP, Bossone CA, Wade CE. Normal physiological values for conscious pigs used in biomedical research. Laboratory Animal Science 1990;40(3):293-8.

14. Jürgens P. New aspects on etiology, biochemistry, and therapy of portal systemic encephalopathy: a critical survey. Nutrition 1997;13(6):560-70.

15. Lockwood AH, McDonald JM, Reiman RE et al. The dynamics of ammonia metabolism in man. Effects of liver disease and hyperammonemia. Journal Of Clinical Investigation 1979:63(3):499-60.

16. Stein TP, Leskiw MJ, Wallace HW. Metabolism of parenterally administered ammonia. Journal Of Surgical Research 1976;21(1):17-20.

17. Almond MK, Smith A, Cohen RD, Iles RA, Flynn G. Substrate and pH effects on glutamine synthesis in rat liver. Biochem J 1991;278(Pt 3):709-14.

18. Atkinson D, Bourke E. Metabolic aspects of the regulation of systemic pH. Am J Physiol 1987;252(6 Pt 2):F947-56.

19. Atkinson D, Camien M. The role of urea synthesis in the removal of metabolic bicarbonate and the regulation of pH. Curr Top Cell Regul 1982;21:261-302.

20. Reaich D, Channon SM, Scrimgeour CM, Goodship TH. Ammonium chloride-induced acidosis increases protein breakdown and amino acid oxidation in humans. American Journal Of Physiology 1992;263(4 Pt 1):E735-9.

21. Roth E, Karner J, Roth-Merten A et al. Effect of alpha-ketoglutarate infusions on organ balances of glutamine and glutamate in anaesthetized dogs in the catabolic state. Clin Sci (Colch) 1991;80(6):625-31.

22. Cynober LA. The use of alpha-ketoglutarate salts in clinical nutrition and metabolic care. Curr Opin Clin Nutr Metab Care 1999; 2(1):33-7.

23. Häussinger D. Hepatocyte heterogeneity in glutamine and ammonia metabolism and the role of an intercellular glutamine cycle during ureogenesis in perfused rat liver. Eur J Biochem 1983;133(2):269-75.

Table 2.2

**Table 1.1.** Dosages effects of $\alpha$-ketoglutaric acid and ammonium chloride on blood gas and hemodynamic variables in Group 1 (n = 8).

| | Group 1 (n=8) | | | | |
| --- | --- | --- | --- | --- | --- |
| | **Baseline** | **Dosage 1** | **Dosage 2** | **Dosage 3** | **Dosage 4** |
| | 0 min | 60 min. | 120 min | 180 min | 240 min |
| **Arterial pH** | $7.46\pm0.01$ | $7.43\pm0.01$ **a** | $7.39\pm0.01$ **ab** | $7.35\pm0.01$ **abc** | $7.30\pm0.01$ **abcd** |
| **Base excess mmol·L$^{-1}$** | $3.9\pm0.5$ | $11.7\pm0.3$ **a** | $-0.5\pm0.3$**ab** | $-2.9\pm0.4$ **abc** | $-5.6\pm0.4$ **abcd** |
| **P$_a$CO$_2$ kPa** | $5.28\pm0.07$ | $5.18\pm0.06$ | $5.37\pm0.06$ **b** | $5.48\pm0.07$ **ab** | $5.44\pm0.07$ |
| **C.O.ml·min$^{-1}$·kg$^{-1}$** | $140\pm10$ | $138\pm17$ | $147\pm17$ | $157\pm16$ | $163\pm17$ |
| **Spl. blood flow ml·min$^{-1}$·kg$^{-1}$** | $51\pm7$ | $43\pm5$ | $44\pm6$ | $54\pm5$ **b** | $49\pm4$ |
| **Leg blood flow ml·min$^{-1}$·kg$^{-1}$** | $6.0\pm0.4$ | $4.7\pm0.4$ **a** | $3.9\pm0.3$ **ab** | $3.5\pm0.2$ **ab** | $3.4\pm0.3$ **ab** |

Values are mean $\pm$ SEM. Statistical significance ($p < 0.05$) according to the Wilcoxon signed rank test is indicated by "**a**" -different from baseline; "**b**" -different from 60 min; "**c**" -different from 120 min; "**d**" -different from 180 min. Dose rates for group 1: Dosage 1 (0-60 min) - NH$_4^+$, 12.3 $\mu$mol·kg$^{-1}$·min$^{-1}$; Dosage 2 (60-120 min) - NH$_4^+$, 12.3 $\mu$mol·kg$^{-1}$·min$^{-1}$ + $\alpha$-ketoglutarate, 2.85 $\mu$mol·kg$^{-1}$·min$^{-1}$; Dosage 3 (120-180 min) - NH$_4^+$, 12.3 $\mu$mol·kg$^{-1}$·min$^{-1}$·h$^{-1}$ + $\alpha$-ketoglutarate, 5.7 $\mu$mol·kg$^{-1}$·min$^{-1}$; Dosage 4 (180-240 min) - NH$_4^+$, 12.3 $\mu$mol·kg$^{-1}$·min

**Table 1.2**. Dosages effects of $\alpha$-ketoglutaric acid and NH$_4$Cl on blood gas and hemodynamic variables in Group 2 ( n = 8).

| | Group2(n=8) | | | | |
| --- | --- | --- | --- | --- | --- |
| | **Baseline**<br>0 min | **Dosage 1**<br>60 min | **Dosage 2**<br>120 min | **Dosage 3**<br>180 min | **Dosage 4**<br>240 min |
| **Arterial pH** | 7.47±0.004 | 7.47±0.01 | 7.42±0.01 **ab** | 7.37±0.01 **abc** | 7.29±0.01 **abcd** |
| **Base excess mmol·L$^{-1}$** | 5.0±0.5 | 4.4±0.31. | 7±0.6 **ab** | -1.8±0.4 **abc** | -6.3±0.4 **abcd** |
| **P$_a$CO$_2$ kPa** | 5.31±0.06 | 5.27±0.06 | .43±0.09 **b** | 5.42±0.15 | 5.42±0.11 |
| **C.O. ml·min$^{-1}$·kg$^{-1}$** | 158±13 | 144±10 **a** | 151±10 | 164±11 | 185±14 **bcd** |
| **Spl. blood flow ml·min$^{-1}$·kg$^{-1}$** | 53±7 | 49±6 | 50±5 | 64±5 **abc** | 73±11 **c** |
| **Leg blood flow ml·min$^{-1}$·kg$^{-1}$** | 7.4±0.8 | 6.0±0.5 a | 4.7±0.3 **ab** | 4.1±0.2 **ab** | 4.3±0.3 **ab** |

Values are mean ± SEM. Statistical significance (p < 0.05) according to the Wilcoxon signed rank test is indicated by "**a**" -different from baseline; "**b**"-different from 60 min; "**c**"-different from 120 min; "d"-different from 180 min. Dose rates for Group 2: Dosage 1 (0-60 min) - $\alpha$-ketoglutarate, 2.85 $\mu$mol·kg$^{-1}$·min$^{-1}$; Dosage 2 (60-120 min) - $\alpha$-ketoglutarate, 2.85 $\mu$mol·kg$^{-1}$·min$^{-1}$ + NH$_4^+$, 12.7 $\mu$mol·kg$^{-1}$·min$^{-1}$; Dosage 3 (120-180 min) - $\alpha$-ketoglutarate, 2.85 $\mu$mol·kg$^{-1}$·min$^{-1}$ + NH$_4^+$, 25.5 $\mu$mol·kg$^{-1}$·min$^{-1}$; Dosage 4 (180-240 min)- $\alpha$-ketoglutarate, 2.85 $\mu$mol·kg$^{-1}$·min$^{-1}$ + NH$_4^+$, 51.0 $\mu$mol·kg$^{-1}$·min$^{-1}$.

**Table 2.1.** Dosages effects of $\alpha$-ketoglutaric acid and ammonium chloride on arterial amino acid, ammonium, urea and glucose concentrations in Group 1 (n = 8).

| | | Group 1 ( n = 8 ) | | | | |
| --- | --- | --- | --- | --- | --- | --- |
| | | **Baseline**<br>0 min | **Dosage 1**<br>60 min | **Dosage 2**<br>120 min | **Dosage 3**<br>180 min | **Dosage 4**<br>240 min |
| **Glutamine** | $\mu$mol·L$^{-1}$ | 384±34 | 450±26**a** | 447±34 | 455±45 | 504±52 |
| **Glutamate** | $\mu$mol·L$^{-1}$ | 181±18 | 191±20 | 198±17 | 209±16 | 213±18 |
| **Alanine** | $\mu$mol·L$^{-1}$ | 503±51 | 412±44**a** | 368±43**ab** | 356±44 **ab** | 344±41**ab** |
| **Arginine** | $\mu$mol·L$^{-1}$ | 71±6 | 80±8 | 96±8 **ab** | 117±7 **abc** | 127±8**abc** |
| **Total AA** | $\mu$mol·L$^{-1}$ | 3186±167 | 3034±139 | 2958±140 | 3080±166 | 3160±165 |
| **NH$_4^+$** | $\mu$mol·L$^{-1}$ | 36±2 | 135±14**a** | 141±13**a** | 145±11 **a** | 115±22**a** |
| **Urea** | mmol·L$^{-1}$ | 3.4±0.4 | 3.4±0.3 | 3.5±0.3 | 3.5±0.2 | 3.6±0.3 |
| **Glucose** | mmol·L$^{-1}$ | 6.0±0.4 | 5.5±0.5 | 4.8±0.6 | 4.6±0.6**a** | 5.2±0.5 **a** |

Values are mean ± SEM. Statistical significance (p < 0.05) according to the Wilcoxon signed rank test is indicated by "**a**" -different from baseline; "**b**"-different from 60 min; "**c**"-different from 120 min; "**d**"-different from 180 min. Dose rates for Group 1: Dosage 1 (0-60 min) - NH$_4^+$, 12.3 $\mu$mol·kg$^{-1}$·min$^{-1}$; Dosage 2 (60-120 min) - NH$_4^+$, 12.3 $\mu$mol·kg$^{-1}$·min$^{-1}$ + $\alpha$-ketoglutarate, 2.85 $\mu$mol·kg$^{-1}$·min$^{-1}$; Dosage 3 (120-180 min) - NH$_4^+$, 12.3 $\mu$mol·kg$^{-1}$·min$^{-1}$·h$^{-1}$ + $\alpha$-ketoglutarate, 5.7 $\mu$mol·kg$^{-1}$·min$^{-1}$; Dosage 4 (180-240 min) - NH$_4^+$, 12.3 mol·kg$^{-1}$·min$^{-1}$ + $\alpha$-ketoglutarate, 11.4 $\mu$mol·kg$^{-1}$·min$^{-1}$.

**Table 2.2.** Dosages effects of $\alpha$-ketoglutaric acid and ammonium chloride on arterial amino acid, ammonium, urea and glucose concentrations in Group 2 ( n = 8).

| | | Group 2(n=8) | | | | |
| --- | --- | --- | --- | --- | --- | --- |
| | | **Baseline**<br>0 min | **Dosage 1**<br>60 min | **Dosage 2**<br>120 min | **Dosage 3**<br>180 min | **Dosage 4**<br>240 min |
| **Glutamine** | $\mu$mol·L$^{-1}$ | 397±26 | 361±24 **a** | 451±27 **ab** | 521±29 **abc** | 621±37 **abcd** |

(continued)

| | | Group 2(n=8) | | | | |
| | | **Baseline**<br>0 min | **Dosage 1**<br>60 min | **Dosage 2**<br>120 min | **Dosage 3**<br>180 min | **Dosage 4**<br>240 min |
|---|---|---|---|---|---|---|
| **Glutamate** | $\mu$mol·L$^{-1}$ | 241$\pm$23 | 264$\pm$32 | 287$\pm$28 **ab** | 284$\pm$25 **a** | 296$\pm$24 **a** |
| **Alanine** | $\mu$mol·L$^{-1}$ | 506$\pm$38 | 462$\pm$34 | 436$\pm$37 | 392$\pm$32 a | 353$\pm$27 **abd** |
| **Arginine** | $\mu$mol·L$^{-1}$ | 77$\pm$8 | 75$\pm$5 | 95$\pm$7**b** | 112$\pm$7**ab** | 128$\pm$6 **abcd** |
| **Total AA** | $\mu$mol·L$^{-1}$ | 3198$\pm$167 | 3044$\pm$158 | 3244$\pm$178 | 3270$\pm$154 | 3233$\pm$119 |
| **NH$_4$$^+$** | $\mu$mol·L$^{-1}$ | 41$\pm$4 | 43$\pm$4 | 147$\pm$10 **ab** | 277$\pm$29 **abc** | 728$\pm$135 **abcd** |
| **Urea** | mmol·L$^{-1}$ | 2.9$\pm$0.2 | 2.7$\pm$0.2 | 2.9$\pm$0.2 **b** | 3.3$\pm$0.2 **bc** | 4.3$\pm$0.2 **abcd** |
| **Glucose** | mmol·L$^{-1}$ | 5.7$\pm$0.5 | 5.6$\pm$0.4 | 5.3$\pm$0.4 | 4.9$\pm$0.5 **b** | 4.7$\pm$0.4 **b** |

Values are mean $\pm$ SEM. Statistical significance ($p < 0.05$) according to the Wilcoxon signed rank test is indicated by "**a**" -different from baseline; "**b**"-different from 60 min; "**c**"-different from 120 min; "**d**"-different from 180 min. Dose rates for Group 2: Dosage 1 (0-60 min) - $\alpha$-ketoglutarate, 2.85 $\mu$mol·kg$^{-1}$·min$^{-1}$; Dosage 2 (60-120 min) - $\alpha$-ketoglutarate, 2.85 $\mu$mol·kg$^{-1}$·min$^{-1}$ + NH$_4$$^+$, 12.7 $\mu$mol·kg$^{-1}$·min$^{-1}$; Dosage 3 (120-180 min) - $\alpha$-ketoglutarate, 2.85 $\mu$mol·kg$^{-1}$·min$^{-1}$+ NH$_4$$^+$, 25.5 $\mu$mol·kg$^{-1}$·min$^{-1}$; Dosage 4 (180-240 min)- $\alpha$-ketoglutarate, 2.85 $\mu$mol·kg$^{-1}$·min$^{-1}$ + NH$_4$$^+$, 51.0 $\mu$mol·kg$^{-1}$·min$^{-1}$.

**Table 3.1.** Turnover effects for different dosages of $\alpha$-ketoglutaric acid and ammonium chloride in Group 1 (n = 8).

| | | Group1 (n=8) | | | | |
| | | **Baseline**<br>0 min | **Dosage 1**<br>60 min | **Dosage 2**<br>120 min | **Dosage 3**<br>180 min | **Dosage 4**<br>240 min |
|---|---|---|---|---|---|---|
| **Glutamine** | splanchnic | 2.52$\pm$0.60 | 2.59$\pm$0.56 | 2.82$\pm$0.32 | 3.50$\pm$0.47 | 3.79$\pm$0.54 |
| | hind leg | -0.04$\pm$0.05 | -0.09$\pm$0.04 | -0.10$\pm$0.05 | -0.14$\pm$0.04 | -0.10$\pm$0.05 |
| **Glutamate** | splanchnic | -5.04$\pm$0.49 | -4.32$\pm$0.28 | -4.33$\pm$0.34 | -4.78$\pm$0.46 | -3.60$\pm$0.25 **abcd** |
| | hind leg | 0.19$\pm$0.03 | 0.22$\pm$0.03 | 0.19$\pm$0.03 | 0.17$\pm$0.03 | 0.16$\pm$0.03 |
| **Alanine** | splanchnic | 3.46$\pm$0.89 | 2.22$\pm$0.28 | .2.73$\pm$0.58 | 3.14$\pm$0.49 | 3.41$\pm$0.73 |
| | hind leg | -0.19$\pm$0.03 | -0.13$\pm$0.04 | -0.21$\pm$0.14 | -0.11$\pm$0.04 | -0.15$\pm$0.05 |
| **Arginine** | splanchnic | 0.06$\pm$0.21 | -0.23$\pm$0.10 | 0.03$\pm$0.15 | 0.11$\pm$0.23 | -0.06$\pm$0.22 |
| | hind leg | 0.01$\pm$0.03 | 0.01$\pm$0.005 | 0.01$\pm$0.02 | 0.01$\pm$0.01 | -0.01$\pm$0.02 |
| **Total AA** | splanchnic | 6.21$\pm$2.54 | 3.93$\pm$2.08 | 4.35$\pm$0.98 | 5.41$\pm$1.43 | 7.19$\pm$2.64 |
| | hind leg | -0.01$\pm$0.32 | 0.12$\pm$0.23 | 0.09$\pm$0.25 | -0.10$\pm$0.16 | -0.10$\pm$0.17 |
| **NH$_4$$^+$** | splanchnic | 0.52$\pm$0.20 | 3.09$\pm$0.28 **a** | 3.34$\pm$0.19 **a** | 4.10$\pm$0.34 **ab** | 2.76$\pm$0.57 **a** |
| | hind leg | 0.02$\pm$0.01 | 0.22$\pm$0.03 **a** | 0.17$\pm$0.02 **a** | 0.13$\pm$0.03ab | 0.05$\pm$0.03 **bcd** |
| **FE(%)-NH$_4$$^+$** | splanchnic | 37$\pm$11 | 81$\pm$4 **a** | 77$\pm$6**a** | 74$\pm$6a | 65$\pm$5 **ab** |
| | hind leg | 12$\pm$6 | 46$\pm$3 **a** | 40$\pm$4 **a** | 34$\pm$3 **a** | 8$\pm$9 **bcd** |
| **Urea** | splanchnic | -3.2$\pm$1.3 | -3.2$\pm$1.4 | -1.1$\pm$1.0 | -6.4$\pm$1.5 **c** | -2.2$\pm$1.6 |
| | hind leg | 0.3$\pm$0.2 | 0.2$\pm$0.2 | 0.6$\pm$0.1 | 0.1$\pm$0.2 | -0.1$\pm$0.3**c** |

Values are mean $\pm$ SEM. Statistical significance ($p < 0.05$) according to the Wilcoxon signed rank test is indicated by "**a**" -different from baseline; "**b**"-different from 60 min; "**c**"-different from 120 min; "**d**"-different from 18p min. Dose rates for Group 1: Dosage 1 (0-60 min) - NH$_4$$^+$, 12.3 $\mu$mol·kg$^{-1}$·min$^{-1}$; Dosage 2 (60-120 min) - NH$_4$$^+$, 12.3 $\mu$mol·kg$^{-1}$·min$^{-1}$ + $\alpha$-ketoglutarate, 2.85 $\mu$mol·kg$^{-1}$·min$^{-1}$; Dosage 3 (120-180 min) - NH$_4$$^+$, 12.3 $\mu$mol·kg$^{-1}$·min$^{-1}$·h$^{-1}$ + $\alpha$-ketoglutarate, 5.7 $\mu$mol·kg$^{-1}$·min$^{-1}$; Dosage 4 (180-240 min) - NH$_4$$^+$, 12.3 $\mu$mol·kg$^{-1}$·min$^{-1}$ + $\alpha$-ketoglutarate, 11.4 $\mu$mol·kg$^{-1}$·min$^{-1}$.

**Table 3.2.** Turnover effects for different dosages of α-ketoglutaric acid and ammonium chloride in Group 2 (n = 8).

| | | Baseline | Dosage 1 | Dosage 2 | Dosage 3 | Dosage 4 |
| | | 0 min | 60 min | 120 min | 180 min | 240 min |
|---|---|---|---|---|---|---|
| | | Group2(n=8) | | | | |
| **Glutamine** | splanchnic | 2.63±0.75 | 2.14±0.31 | 2.13±0.62 | 3.82±0.61 **c** | 5.48±1.32**bc** |
| | hind leg | -0.15±0.11 | -0.09±0.07 | -0.17±0.04 | -0.24±0.05 | -0.19±0.07 |
| **Glutamate** | splanchnic | -6.58±0.52 | -5.83±0.61 | -5.23±0.67 | -5.94±0.79 | -6.16±1.61 |
| | hind leg | 0.30±0.05 | 0.25±0.06 | 0.34±0.05 | 0.27±0.03 **c** | 0.28±0.05 |
| **Alanine** | splanchnic | 4.24±1.14 | 4.11±0.97 | 2.79±0.68 | 3.65±0.52 | 3.85±0.84 |
| | hind leg | -0.27±0.13 | -0.20±0.09 | -0.17±0.03 | -0.15±0.04 | -0.15±0.04 |
| **Arginine** | splanchnic | 0.39±0.36 | 0.26±0.22 | 0.03±0.15 | -0.45±0.41 | -0.04±0.25 |
| | hind leg | 0.09±0.04 | 0.03±0.02 | 0.02±0.02 | -0.05±0.04 **abc** | 0.02±0.04 **d** |
| **Total AA** | splanchnic | 7.58±4.12 | 6.18±2.26 | 2.75±4.43 | 5.11±2.98 | 9.84±5.41 |
| | hind leg | -0.23±0.82 | -0.36±0.64 | 0.16±0.28 | -0.11±0.21 | -0.29±0.35 |
| **NH$_4$$^+$** | splanchnic | 0.53±0.22 | 0.50±0.21 | 3.72±0.28 **ab** | 9.73±0.57 **abc** | 19.59±2.98 **abcd** |
| | hind leg | 0.04±0.03 | 0.01±0.02 | 0.23±0.03 **ab** | 0.31±0.04 **abc** | 0.51±0.14 **abc** |
| **FE(%)-NH$_4$$^+$** | splanchnic | 27±13 | 28±10 | 70±4 ab | 77±4 **ab** | 56±7 **bd** |
| | hind leg | 8±11 | 5±7 | 43±4 ab | 35±2 **b** | 20±3 **bcd** |
| **Urea** | splanchnic | -6.0±3.2 | -4.0±1.2 | -3.0±2.8 | -2.6±3.3 | 5.3±4.0 |
| | hind leg | -0.3±0.6 | -0.2±0.4 | 0.2±0.4 | 0.5±0.2 | 0.7±0.2 **b** |

Values are mean ± SEM. Statistical significance (p < 0.05) according to the Wilcoxon signed rank test is indicated by "**a**" -different from baseline; "**b**"-different from 60 min; "**c**"-different from 120 min; "**d**"-different from 180 min. Dose rates for Group 2: Dosage 1 (0-60 min) - α-ketoglutarate, 2.85 μmol·kg$^{-1}$·min$^{-1}$; Dosage 2 (60-120 min) - α-ketoglutarate, 2.85 mol·kg$^{-1}$·min$^{-1}$ + NH$_4$$^+$, 12.7 μmol·kg$^{-1}$·min$^{-1}$; Dosage 3 (120-180 min) - α-ketoglutarate, 2.85 μmol·kg$^{-1}$·min$^{-1}$ + NH$_4$$^+$, 25.5 μmol·kg$^{-1}$·min$^{-1}$; Dosage 4 (180-240 min)- α-ketoglutarate, 2.85 μmol·kg$^{-1}$·min$^{-1}$ + NH$_4$$^+$, 51.0 μmol·kg$^{-1}$·min$^{-1}$.

**Claims**

1. Use of a pair of pharmaceutical agents selected from (a) at least one of α-ketoglutarate and α-ketoglutaric acid and (b) ammonium, the amounts of the pair being effective to preserve skeletal muscle for the manufacture of a medicament for their concomitant administration in separate pharmaceutically acceptable carriers for preserving bodily protein stores in a catabolic patient.

2. The use of claim 1, wherein the administration of (a) or (b) or both lasts for more than one hour.

3. The use of claim 1, wherein the administration of ammonium lasts for more than one hour.

4. The use of claim 2, wherein the concomitant administration lasts for more than 6 hours but less than 36 hours.

5. The use of claim 1, wherein the administration is to a patient having undergone accidental trauma or surgery and the administration is intermittent or continuous for at least three days during the posttraumatic/postoperative period in which the patient is in a catabolic state.

6. The use of claim 1, wherein administration is by infusion.

7. The use of claim 6, wherein the amount of infusion administered of (a) is from 0.02 μmol·kg$^{-1}$ ·min$^{-1}$ to 30 μmol·kg$^{-1}$ ·min$^{-1}$.

8. The use of claim 7, wherein the amount of infusion administered of (a) is from 0.5 μmol·kg$^{-1}$ ·min$^{-1}$ to 15 μmol·kg$^{-1}$ ·min$^{-1}$.

9. The use of claim 6, wherein the amount of infusion administered of $NH_4^+$ is from 0.5 $\mu$mol·kg$^{-1}$·min$^{-1}$ to 20 $\mu$mol·kg$^{-1}$·min$^{-1}$.

10. The use of claim 9, wherein the amount of infusion administered of $NH_4^+$ is from 1 $\mu$mol·kg$^{-1}$·min$^{-1}$ to 10 $\mu$mol·kg$^{-1}$·min$^{-1}$.

11. The use of claim 9, wherein the amount of infusion administered of $NH_4^+$ is increased over the period of administration.

12. The use of claim 11, wherein the amount of infusion administered of (a) is from 0.02 $\mu$mol·kg$^{-1}$·min$^{-1}$ to 30 $\mu$mol·kg$^{-1}$·min$^{-1}$.

13. The use of claim 11, wherein said increase is by a factor of from 1.5 to 8.

14. The use of claim 13, wherein said increase is by a factor of from 2 to 5.

15. The use of claim 1, wherein the ammonium is ammonium chloride.

16. A pharmaceutical dosage unit comprising a first pharmaceutical composition comprising at least one of $\alpha$-ketoglutarate and $\alpha$-ketoglutaric acid in a pharmaceutically acceptable carrier and a second pharmaceutical composition comprising ammonium in a pharmaceutically acceptable carrier, the total amount of the at least one of $\alpha$-ketoglutarate and $\alpha$-ketoglutaric acid and the ammonium being effective to preserve skeletal muscle.

17. The dosage unit of claim 16, wherein said amount of at least one $\alpha$-ketoglutarate and $\alpha$-ketoglutaric acid is for infusion at a rate of from 0.02 $\mu$mol·kg$^{-1}$·min$^{-1}$ to 30 $\mu$mol·kg$^{-1}$·min$^{-1}$ and said amount of $NH_4^+$ is for infusion at a rate of from 0.5 $\mu$mol·kg$^{-1}$·min$^{-1}$ to 20 $\mu$mol·kg$^{-1}$·min$^{-1}$.

18. The unit of claim 16, wherein both carriers are infusion carriers.

19. The unit of claim 16, wherein both carriers are oral carriers.

20. The unit of claim 16, wherein $\alpha$-ketoglutarate is in form of its sodium salt.

21. The unit of claim 16, wherein ammonium is ammonium chloride.

**Patentansprüche**

1. Verwendung eines Paars pharmazeutischer Mittel ausgewählt unter (a) mindestens einem von $\alpha$-Ketoglutarat und $\alpha$-Ketoglutarsäure und (b) Ammonium, wobei die Mengen des Paars zur Aufrechterhaltung des Skelettmuskels wirksam sind, zur Herstellung eines Medikaments, für deren gleichzeitige Verabreichung in getrennten, pharmazeutisch annehmbaren Trägem, zur Aufrechterhaltung von Körperprotein-Speichern in einem katabolischen Patienten.

2. Verwendung nach Anspruch 1, wobei die Verwendung von (a) oder (b) oder beiden mehr als eine Stunde andauert.

3. Verwendung nach Anspruch 1, wobei die Verabreichung von Ammonium für mehr als eine Stunde andauert.

4. Verwendung nach Anspruch 2, wobei die gleichzeitige Verabreichung mehr als 6 Stunden, jedoch weniger als 36 Stunden andauert.

5. Verwendung nach Anspruch 1, wobei die Verabreichung an einem Patienten erfolgt, der ein Unfalltrauma oder eine Operation erfahren hat, und wobei die Verabreichung unterbrochen oder kontinuierlich für mindestens drei Tage während der posttraumatischen/postoperativen Zeitspanne, in dem sich der Patient in einem katabolischen Zustand befindet, erfolgt.

6. Verwendung nach Anspruch 1, wobei die Verabreichung mittels Infusion erfolgt.

7. Verwendung nach Anspruch 6, wobei die Menge an an (a) verabreichter Infusion von 0,02 $\mu$mol/kg x min bis 30

$\mu$mol/kg x min ist.

**8.** Verwendung nach Anspruch 7, wobei die Menge an an (a) verabreichter Infusion von 0,5 $\mu$mol/kg x min bis 15 $\mu$mol/kg x min ist.

**9.** Verwendung nach Anspruch 6, wobei die Menge an NH4$^+$ verabreichter Infusion von 0,5 $\mu$mol/kg x min bis 20 $\mu$mol/kg x min ist.

**10.** Verwendung nach Anspruch 9, wobei die Menge an NH4$^+$ verabreichter Infusion von 1 $\mu$mol/kg x min bis 10 $\mu$mol/kg x min ist.

**11.** Verwendung nach Anspruch 9, wobei die Menge an NH4$^+$ verabreichter Infusion über die Zeitspanne der Verabreichung erhöht wird.

**12.** Verwendung nach Anspruch 11, wobei die Menge an an (a) verabreichter Infusion von 0,02 $\mu$mol/kg x min bis 30 $\mu$/kg x min ist.

**13.** Verwendung nach Anspruch 11, wobei der Anstieg um einen Faktor von 1,5 bis 8 erfolgt.

**14.** Verwendung nach Anspruch 13, wobei der Anstieg um einen Faktor von 2 bis 5 erfolgt.

**15.** Verwendung nach Anspruch 1, wobei das Ammonium Ammoniumchlorid ist.

**16.** Pharmazeutische Dosierungseinheit, welche eine erste pharmazeutische Zusammensetzung umfasst, die mindestens eines von $\alpha$-Ketoglutarat und $\alpha$-Ketoglutarsäure in einem pharmazeutisch annehmbaren Träger enthält, und eine zweite pharmazeutische Zusammensetzung umfasst, welche Ammonium in einem pharmazeutisch annehmbaren Träger enthält, wobei die Gesamtmenge von mindestens einem von $\alpha$-Ketoglutarat und $\alpha$-Ketoglutarsäure und Ammonium wirksam ist, um den Skelettmuskel zu erhalten.

**17.** Dosierungseinheit nach Anspruch 16, worin die Menge von mindestens einem von $\alpha$-Ketoglutarat und $\alpha$-Ketoglutarsäure für eine Infusion bei einer Menge von 0,2 $\mu$mol/kg x min bis 30 $\mu$mol/kg x min ist, und worin die Menge an NH4$^+$ für eine Infusion in einer Geschwindigkeit von 0,5 $\mu$mol/kg x min bis 20 $\mu$mol/kg x min ist.

**18.** Einheit nach Anspruch 16, worin beide Träger Infusionsträger sind.

**19.** Einheit nach Anspruch 16, worin beide Träger orale Träger sind.

**20.** Einheit nach Anspruch 16, worin das $\alpha$-Ketoglutarat in Form dessen Natriumsalzes vorliegt.

**21.** Einheit nach Anspruch 16, worin das Ammonium Ammoniumchlorid ist.


**Revendications**

**1.** Utilisation d'une paire d'agents pharmaceutiques choisie dans (a) au moins un parmi l'$\alpha$-cétoglutarate et l'acide $\alpha$-cétoglutarique et (b) l'ammonium, les quantités de la paire étant efficaces pour conserver le muscle squelettique pour la fabrication d'un médicament pour leur administration concomitante dans des véhicules pharmaceutiquement acceptables distincts pour conserver des réserves de protéines corporelles chez un patient catabolique.

**2.** Utilisation selon la revendication 1, dans laquelle l'administration de (a) ou de (b) ou des deux dure plus d'une heure.

**3.** Utilisation selon la revendication 1, dans laquelle l'administration de l'ammonium dure plus d'une heure.

**4.** Utilisation selon la revendication 2, dans laquelle l'administration concomitante dure plus de 6 heures mais moins de 36 heures.

**5.** Utilisation selon la revendication 1, dans laquelle l'administration concerne un patient ayant subi un traumatisme accidentel ou une intervention chirurgicale, et l'administration est intermittente ou continue durant au moins trois jours durant la période post-traumatique/post-opératoire pendant laquelle le patient se trouve dans un état catabolique.

**6.** Utilisation selon la revendication 1, dans laquelle l'administration se fait par perfusion.

**7.** Utilisation selon la revendication 6, dans laquelle la quantité administrée par perfusion de (a) est de 0,02 à 30 $\mu$moles.kg$^{-1}$.min$^{-1}$.

**8.** Utilisation selon la revendication 7, dans laquelle la quantité administrée par perfusion de (a) est de 0,5 à 15 $\mu$moles.kg$^{-1}$.min$^{-1}$.

**9.** Utilisation selon la revendication 6, dans laquelle la quantité administrée par perfusion de $NH_4^+$ est de 0,5 à 20 $\mu$moles.kg$^{-1}$.min$^{-1}$.

**10.** Utilisation selon la revendication 9, dans laquelle la quantité administrée par perfusion de $NH_4^+$ est de 1 à 10 $\mu$moles.kg$^{-1}$.min$^{-1}$.

**11.** Utilisation selon la revendication 9, dans laquelle la quantité administrée par perfusion de $NH_4^+$ est augmentée durant la période de perfusion.

**12.** Utilisation selon la revendication 11, dans laquelle la quantité administrée par perfusion de (a) est de 0,02 à 30 $\mu$moles.kg$^{-1}$.min$^{-1}$.

**13.** Utilisation selon la revendication 11, dans laquelle ladite augmentation est d'un facteur 1,5 à 8.

**14.** Utilisation selon la revendication 13, dans laquelle ladite augmentation est d'un facteur 2 à 5.

**15.** Utilisation selon la revendication 1, dans laquelle l'ammonium est le chlorure d'ammonium.

**16.** Dose unitaire pharmaceutique comprenant une première composition pharmaceutique comprenant au moins un parmi l'$\alpha$-cétoglutarate et l'acide $\alpha$-cétoglutarique dans un véhicule pharmaceutiquement acceptable et une deuxième composition pharmaceutique comprenant de l'ammonium dans un véhicule pharmaceutiquement acceptable, la quantité totale d'au moins un parmi l'$\alpha$-cétoglutarate et l'acide $\alpha$-cétoglutarique et l'ammonium étant efficace pour conserver le muscle squelettique.

**17.** Dose unitaire selon la revendication 16, dans laquelle ladite quantité d'au moins un parmi l'$\alpha$-cétoglutarate et l'acide $\alpha$-cétoglutarique est destiné à une perfusion à une vitesse de 0,02 à 30 $\mu$moles.kg$^{-1}$.min$^{-1}$, et ladite quantité de $NH_4^+$ est destinée à une infusion à une vitesse de 0,5 à 20 $\mu$moles.kg$^{-1}$.min$^{-1}$.

**18.** Dose unitaire selon la revendication 16, dans laquelle les deux véhicules sont des véhicules de perfusion.

**19.** Dose unitaire selon la revendication 16, dans laquelle les deux véhicules sont des véhicules oraux.

**20.** Dose unitaire selon la revendication 16, dans laquelle l'$\alpha$-cétoglutarate est sous la forme de son sel de sodium.

**21.** Dose unitaire selon la revendication 16, dans laquelle l'ammonium est le chlorure d'ammonium.

**GROUP 1**

Stabilization period

$NH_4^+$
12.3 $\mu mol\cdot kg^{-1}\cdot min^{-1}$

a-ketoglutaric acid
2.85 $\mu mol\cdot kg^{-1}\cdot min^{-1}$

a-ketoglutaric acid
5.7 $\mu mol\cdot kg^{-1}\cdot min^{-1}$

a-ketoglutaric acid
11.4 $\mu mol\cdot kg^{-1}\cdot min^{-1}$

Time (min)

-60     0     60     120     180     240

Baseline

**GROUP 2**

Stabilization period

a-ketoglutaric acid
2.85 $\mu mol\cdot kg^{-1}\cdot min^{-1}$

$NH_4^+$
12.7 $\mu mol\cdot kg^{-1}\cdot min^{-1}$

$NH_4^+$
25.5 $\mu mol\cdot kg^{-1}\cdot min^{-1}$

$NH_4^+$
51.0 $\mu mol\cdot kg^{-1}\cdot min^{-1}$

Time (min)

-60     0     60     120     180     240

Baseline

*Fig. 1*

EP 1 363 615 B1

*Fig. 2*

EP 1 363 615 B1

Fig. 3

Fig. 4